# EUROPEAN PATENT SPECIFICATION

(11) **EP 1 951 330 B1**
(45) Date of publication and mention of the grant of the patent: **23.12.2009**
(21) Application number: 06849268.5
(22) Date of filing: 08.11.2006
(51) Int. Cl.: A61L 27/34, A61L 31/10, A61L 27/50, C10M 111/04

(54) **LUBRICIOUS COMPOUND AND MEDICAL DEVICE MADE OF THE SAME**
SCHMIERSTOFFVERBINDUNG UND DARAUS HERGESTELLTES MEDIZINISCHES INSTRUMENT
COMPOSÉ LUBRIFIANT ET DISPOSITIF MÉDICAL CONSTITUÉ DE CELUI-CI

(30) Priority: 15.11.2005 US 274544
(43) Date of publication of application: 06.08.2008
(73) Proprietor: Cardiac Pacemakers, Inc., St. Paul MN 55112 (US)
(72) Inventor: BAVARO, Vincent, P., Temecula, California 92592 (US)
(74) Representative: Pfenning, Meinig & Partner GbR
(86) International application number: PCT/US2006/060668
(87) International publication number: WO 2007/081603

(56) References cited:
- US-A- 5 041 100
- US-A1- 2003 109 628
- US-A1- 2005 055 044

## Description

### FIELD OF THE INVENTION

The present invention relates to hydrophilic polymers. More particularly, the present invention relates to a hydrophilic lubricous polymer blend with an improved resistance to being physically abraded from a surface.

### BACKGROUND OF THE INVENTION

Water-sensitive hydrophilic polymers are commonly used in the manufacture of various personal care and medical devices. The water-sensitive polymers function to provide lubricity to the device when it becomes wetted with an aqueous solution such as water or a body fluid. The water-sensitive polymers may be used in conjunction with water-insoluble polymers that function to provide the appropriate structural characteristics and mechanical integrity to the device for its intended use. Typical medical devices that can benefit from lubricious properties include, for example, catheters, guide wires, endotracheal tubes and implants.

Patents have reported coating medical devices with water-soluble polymers that are hydrophilic. Such hydrophilic coatings have also been referred to as lubricous or "slippery" coatings. Typically, the hydrophilic polymer is dissolved in a suitable solvent and then applied to the desired medical device. The solvent is then evaporated to yield the coating. Oven drying may be utilized to remove the solvents. When the hydrophilic material is coated on the surface utilizing solvents in a wet method the polymer is usually formed as a fairly thin layer. The hydrophilic coating may break down or be removed upon prolonged turbulent flow,* mechanical abrasion or soaking. Other drawbacks to the solution coating and curing process approach may include solution pot life, coating thickness control, and durability. See, for example, U.S. Pat. Nos. 4,119,094, 5,077,352 and 5,091,205, and EP Patent Nos. 0 106 004 B1 and 0 166 998 B1.
U.S. patent application publication US 2003/0109628 A1 relates to liquid absorbing thermoplastic materials usable as absorbent materials for disposable absorbent articles. The liquid absorbing thermoplastic materials may comprise a) thermoplastic compositions comprising thermoplastic polymers and suitable compatible plasticizers and b) superabsorbent articles dispersed in the thermoplastic compositions.
U.S. patent application publication US 2005/0055044 A1 relates to an ultraviolet curable lubricious coating including at least one lubricious polymer and at least one oxygen-insensitive cross linkable polymer, methods of making and using the same and articles coated therewith.

U.S. Pat. No. 5,061,424 discloses a method for preparing a shaped medical device provided with a lubricous coating. A coating composition comprising a blend of polyurethane and polyvinylpyrrolidone and polyethylene glycol is co-extruded with a substrate polymer to give a shaped medical device having a layer of the coating composition that then becomes lubricous when contacted with water.

U.S. Pat. No. 5,041,100 discloses a method for coating a substrate with a solution of polyethylene oxide and polyurethane. The polyethylene oxide is mixed with the polyurethane. The blend is then formed into a solution and then applied to medical device and dried to form a coating.

U.S. Pat. Nos. 5,113,585 and 5,454,164 report polymer blends for utilization in shaving systems. The polymer blends taught in these patents are specifically designed to abrade off with use in order to provide for skin lubrication.

Accordingly, there is a need in the art for improved lubricious polymer materials for incorporation into medical devices.

### BRIEF SUMMARY OF THE INVENTION

The present invention includes a blend of two or more polymer materials including a water insoluble polymer and a hydrophilic water-soluble polymer, the polymer blend is a finely dispersed blend that provides a lubricious surface.

Another embodiment of the present invention includes a method of forming a medical device from a lubricious polymer, the steps including drying a polyethylene oxide of a molecular weight between about 200,000 and about 7,000,000, drying a polyether block amide, melt mixing the polymers into a generally uniform blend by feeding the polymers at a desired rate into a compounding extruder, forming the blend into a desired medical device, and cross-linking the polymer blend.

Another embodiment may be a lubricious polymer blend coating with an improved resistance to abrasion including a water soluble polymer melt mixed with a water insoluble polymer to form a polymer blend, the polymer blend coated onto a desired surface and cross-linked by exposure to a desired amount of radiation.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 illustrates a scanning electron microscope digital image of a polymer blend of the present invention.

FIG. 2 illustrates a scanning electron microscope digital image a prior art polymer blend.

FIG. 3A illustrates a two-layer tube made with the present invention polymer blend.

FIG. 3B illustrates another two-layer tube made with the present invention polymer blend.

FIG. 4 illustrates the swell characteristics of a tube made with the present invention polymer blend.

FIG. 5A illustrates three-layer tube made with the present invention polymer blend.

FIG. 5B illustrates another three-layer tube made with the present invention polymer blend.

FIG. 5C illustrates yet another three-layer tube made with the present invention polymer blend.

FIG. 6 illustrates a comparison of the frictional force of two embodiments of the present invention against silicone versus prior art lubricious coatings.

FIG. 7 illustrates a comparison of the frictional force of two embodiments of the present invention against polyurethane versus prior art lubricious coatings.

FIG. 8 illustrates the swell rate of a tube made with an alternative embodiment polymer blend of the present invention.

FIG. 9 illustrates the force required to move a silicone tipped PU lead through a catheter made with a polyblend.

FIG. 10 illustrates the force required to move a silicone tipped PU lead through a catheter made with a polyblend and cross-linked.

FIG. 11 is a spectrograph of a polyblend extract.

FIG. 12 is a graph showing the amount of material extracted from the cross-linked polyblend versus the amount of cross-linking radiation utilized.

FIG. 13 is a picture showing the cloud point of various polyblend extracts cross-linked with different amounts of radiation.

FIG. 14 illustrates the percent weight loss of material for various polyblend materials.

FIG. 15 illustrates the percent weight loss of material for various polyblend materials.

### DETAILED DESCRIPTION OF THE INVENTION

The present invention is a formulation for and method of making a lubricious hydrophilic polymer blend and medical devices incorporating the same. The lubricious hydrophilic polymer blend may be referred to as a "polyblend," a "hydrophilic polyblend," or a "lubricious polymer." In addition, the polyblend may be referred to as a hydrogel after cross-linking and exposure to a suitable aqueous solvent. A hydrogel is a colloidal gel in which the particles are dispersed in an aqueous solvent but only loses little or none of its structure to the solution.

The lubricious hydrophilic polyblend includes a lubricious water soluble polymer and an insoluble polymer. The two polymers are melt mixed and solidified to form a finely dispersed polyblend. The present invention polyblend can be utilized to provide a lubricious coating on a medical device formed by extruding, co-extruding, injection molding, or die forming, or, in further embodiments, the polyblend can be directly coated on a medical device. Embodiments of the coating formed using the lubricious hydrophilic polyblend of the present invention may be more robust and allow for superior permanence compared to previously taught lubricious coatings. As may be appreciated, the lubricious hydrophilic polyblend may be formed with or on any type of underlying structural article or framework to impart the desired lubricious properties to the final product.

Medical devices such as guidewires, catheters, sheaths, tubes, etc. that incorporate the present invention polyblend may help to reduce damage to the body during insertion because the lubricious surface will exert reduced frictional forces. Such medical devices may also help to reduce blood clotting as well. The materials of the present invention, therefore, help to prevent the devices from locking up or sticking during delivery procedures. In addition, the lubricious polyblend is not released or abraded away during use because the lubricious polymer is captured in the structural (or matrix) polymer.

The selection of the lubricious water-soluble polymer may depend on a number of factors. The lubricious polymer is partially miscible in the structural polymer but not completely miscible. When the lubricious polymer is only partially miscible rather than completely miscible the final lubricious hydrophilic polyblend will retain pockets of lubricious material dispersed throughout the polyblend. The lubricious polymer may also have a lower melting point and therefore a lower viscosity at a given temperature than the structural polymer. The lower viscosity lubricious polymer is more likely to migrate towards the outer surface of the polyblend. A lubricious hydrophilic material may be able to absorb many times its own weight in water.

In addition, the molecular weight may affect the lubricity of the final compound and so may be a factor in polymer selection. Extrusion grade resins may be of a higher molecular weight and therefore have more melt strength and will have more easily processed melt flow properties.

A lubricious hydrophilic polymer includes polyethylene oxide (PEO). Other lubricious materials may also be incorporated, such as polypropylene oxide (PPO), polyethylvinylalcohol (EVOH), polyethylvinylacetate (EVA), polyvinylpyrolidone (PVP), and other water-soluble lubricious polymers known to those skilled in the art may also be incorporated.

Structural polymers may include polyamides, polyurethanes, polyesters, olefin derived copolymers, polyethylene, high-density polyethylene (HDPE), natural and synthetic rubbers, styrenics, thermoplastic elastomers, and other specialty polymers. Polyamides may include homopolymers and copolymers like Nylon® 12 and 11, Pebax®, and Vestamid® resins. Nylon® 11 and Nylon® 12 copolymers may range in shore hardness from about 80D to 25D. Pebax is a polyether block amide manufactured by Arkema, Philadelphia, Pa. and is available in a variety of durometers. Polyurethanes may include polyesterurethanes and polyetherurethanes, like Pellethane® or Texin. One structural polymer may include polyetherurethane with a shore hardness from about 75D to 90D. Polyesters may include polyethylene terephthalate, polybutylene terephthalate, and co-polyesters like Hytrel® and Arnitel®. Rubbers may include silicone or Santoprene®. Thermoplastic elastomers may include commercially available materials like Kraton®.

In certain embodiments the structural polymer may be cross-linked by a predetermined amount to control the hydration rates and the swell of the polyblend. In further embodiments stabilizers may be included in the hydrophilic polymer blend, such as, for example, Irganox B225 or 1098. The polymer blend may also be formulated to include other advantageous materials, such as stabilizers, drugs, mixing aids, flow aids, plasticizers, heat stabilizers, antimicrobial agents, etc. In further embodiments, other anti-oxidants or other types of additives may also be utilized.

One hydrophilic polyblend of the present invention may include up to 30% or about 30 to about 60% PEO by weight. The polyblend may furthermore contain 35-50%, 40-50% PEO, or, particularly, about 40% PEO. The PEO is preferably greater than 100,000 MW. The PEO may include a molecular weight of about 200,000 to about 7,000,000, more particularly about 500,000 to 2,000,000, or, more particularly, about 1,000,000. In still further embodiments, the polyblend may be diluted during the extrusion or other medical device forming process to form materials with a lower weight percent of the hydrophilic polymer.

**EXAMPLES**

Example 1

A PEO with a molecular weight of 7,000,000(Dow WSR 303) was selected as the hydrophilic polymer and Pebax 72D was selected for the structural polymer. The final hydrophilic polymer blend included PEO at 40% by weight.

| **Step** | **Setting** |
|---|---|
| **Resin Drying Process** | |
| Drying Temp | 170°F (Pebax)/60°C (PEO) |
| Drying Time | 4 hrs. |

| **Feeder Parameters** | |
|---|---|
| PEO ( 7 million MW) | 70 grams/minute |
| Pebax 72D | 93 grams/minute |

| **Extruder Parameters** | |
|---|---|
| Zone #1 | 320°F |
| Zone #2 | 355°F |
| Zone #3 | 375°F |
| Zone #4 | 375°F |
| Die Zone (#5) | 375°F |
| **Screw Speed** | 200 rpm |

| **Extruder Responses** | |
|---|---|
| Drive Torque | 72% |
| Extruder Output | 22 lbs./hr |
| Die Pressure | 350 psi |
| **Melt Temp.** | 373°F |

| **Process Water Temps** | |
|---|---|
| Roll Temp | 45°F |
| Chiller Temp | 45°F |

| **Pelletizing Process** | |
|---|---|
| Pelletizer Speed | 210 rpm |

The polyether block amide was first dried at 170 °F for four hours. The PEO was dried at 60 °C in a vacuum oven (<25 mbar) for four hours. The drying time in the present example and all of the examples below can be for about the listed time or longer. The polymer materials were then separately loaded into two feeders controlled by a feeder control for addition to the compounding extruder. The compounding extruder was a Wemer and Pfliedere ZSK30 co-rotating twin screw extruder. The extruder was equipped with a low shear/low energy screw that included two mixing zones, one dispersive and one distributive, each with six diameters of elements. The aspect ratio of the selected screw was 30:1 length:diameter and included modular conveying and mixing elements.

The PEO feeder was set at 70 grams/minute and the Pebax feeder was set at 93 grams/minute. The mixing barrel included four heat zones. The various heat zones and the screw type and rate allowed the two material feedstreams to be mixed and homogenized before it was passed through the die. The temperature zones of the barrel ranged from 320 to 375 °F. The extruder response had a drive torque of 72% and the extruder output was 22 pounds per hour. The die temperature was set at 375 °F and the die pressure response was 350 psi. The die temperature zone and pressure can be controlled to insure a desired strand viscosity. The extensional viscosity (i.e., melt strength) of the material when it passed through the die was adjusted so that the produced polyblend strand maintained its shape until it was properly cooled.

In the present embodiments a die with four holes of 0.180" inch diameter was utilized to form the polyblend into four parallel strands. The extruded polyblend strands were then drawn out and cooled on the chill roller. Each strand was drawn to 0.100" before being pelletized. A cooled water/glycol solution (1:1) chilled each roller but in the present embodiment no water touched the hydrophilic polyblend during the cooling process. The chill rolls utilized were Davis Standard laboratory grade three-roll stack sheet extrusion rollers. The pelletizer was a Gala strand pelletizer.

Once the molten strands were solidified the polymer blend was chopped/cut into pellets by the pelletizer set at 210 rpm.

Example 2

A PEO with a molecular weight of 7,000,000 was utilized as the hydrophilic polymer and Pebax 72D was selected as the structural polymer. The final hydrophilic polymer blend included PEO at 40% by weight.

| **Step** | **Setting** |
|---|---|
| **Resin Drying Process** | |
| Drying Temp | PEO - 50°C with vacuum Pebax - 160°F with desiccant forced air |
| Drying Time | 12 hrs |

| **Feeder Parameters** | |
|---|---|
| PEO (7 million MW) | 50 grams/minute |
| Pebax 72D | 75 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | 152 rpm |

| **Extruder Responses** | |
|---|---|
| Drive Torque | 79% |
| Extruder Output | 16.5 Ibs./hr |
| Die Pressure | 500 psi |

| **Process Water Temps** | |
|---|---|
| Roll Temp | 32°F |
| Chiller Temp | 32°F |

| **Pelletizing Process** | |
|---|---|
| Pelletizer Speed | 160 rpm |

Example 3

The next example utilized a PEO with a molecular weight of 7,000,000 and Pebax 72D. The final hydrophilic polymer blend included PEO at 60% by weight

| **Step** | **Setting** |
|---|---|
| **Resin Drying Process** | |
| Drying Temp | PEO - 50°C with vacuum Pebax - 160°F with desiccant forced air |
| Drying Time | 24 hrs / 24 hrs |

| **Feeder Parameters** | |
|---|---|
| PEO (7 million MW) | 75.0 grams/minute |
| Pebax 72D | 50.0 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | 175 rpm |

| **Extruder Responses** | |
|---|---|
| Drive Torque | 81% |
| Extruder Output | 16.5 Ibs./hr |
| Die Pressure | 680 psi |

| **Process Water Temps** | |
|---|---|
| Roll Temp | 32°F |
| Chiller Temp | 32°F |

| **Pelletizing Process** | |
|---|---|
| Pelletizer Speed | 130 rpm |

Example 4

A PEO with a molecular weight of 1,000,000 (Dow WSR N12K) was utilized as the hydrophilic polymer and Pebax 72D was the structural polymer. The final hydrophilic polymer blend included PEO at 40% by weight.

| **Step** | **Setting** |
|---|---|
| **Resin Drying Process** | |
| Drying Temp | PEO - 50°C with vacuum Pebax - 160°F with desiccant forced air |
| Drying Time | 12 hrs |

| **Feeder Parameters** | |
|---|---|
| PEO (1,000,000 MW) | 50 grams/minute |
| Pebax 72D | 75 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | 175 rpm |

| **Extruder Responses** | |
|---|---|
| Drive Torque | 65% |
| Extruder Output | 16.5 Ibs./hr |
| Die Pressure | 420 psi |

| **Process Water Temps** | |
|---|---|
| Roll Temp | 32°F |
| Chiller Temp | 32°F |

| **Pelletizing Process** | |
|---|---|
| Pelletizer Speed | 120 rpm |

Example 5

A PEO with a molecular weight of 200,000 (Dow WSR N80) was mixed with Pebax 72D. The final hydrophilic polymer blend included PEO at 40% by weight.

| **Step** | **Setting** |
|---|---|
| **Resin Drying Process** | |
| Drying Temp | PEO - 50°C with vacuum Pebax - 160°F with desiccant forced air) |
| Drying Time | 24 hrs / 24 hrs |

| **Feeder Parameters** | |
|---|---|
| PEO (200,000 MW) | 50.0 grams/minute |
| Pebax 72D | 75.0 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | 173 rpm |

| **Extruder Responses** | |
|---|---|
| Drive Torque | 58% |
| Extruder Output | 16.5 Ibs./hr |
| Die Pressure | 250 psi |
| **Melt Temp.** | 352°F |

| **Process Water Temps** | |
|---|---|
| Roll Temp | 32°F |
| Chiller Temp | 33°F |

| **Pelletizing Process** | |
|---|---|
| Pelletizer Speed | 125 rpm |

Example 6

The hydrophilic polymer was a PEO with a molecular weight of 1,000,000 and the structural polymer was Pebax 72D. The final hydrophilic polymer blend included PEO at 40% by weight.

| **Step** | **Setting** |
|---|---|
| **Resin Drying Process** | |
| Drying Temp | PEO - 50°C with vacuum Pebax - 160°F with desiccant forced air |
| Drying Time | 36 hrs / 24 hrs |

| **Feeder Parameters** | |
|---|---|
| PEO (1,000,000 MW) | 50.0 grams/minute |
| Pebax 72D | 75.0 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | 170 rpm |

| **Extruder Responses** | |
|---|---|
| Drive Torque | 70% |
| Extruder Output | 16.5 lbs. /hr |
| Die Pressure | 520 psi |
| **Melt Temp.** | 341°F |

| **Process Water Temps** | |
|---|---|
| Roll Temp | 32°F |
| Chiller Temp | 33°F |

| **Pelletizing Process** | |
|---|---|
| Pelletizer Speed | 125 rpm |

Example 7

A PEO of with a molecular weight of 7,000,000 was mixed with HDPE as the structural polymer. The HDPE was a Quantum HDPE 6007 (0.6 MFI) (Phillips Slurry process). The final hydrophilic polymer blend included PEO at 35% by weight. Only the PEO was dried as the HDPE is hydrophobic. The PEO was dried under a vacuum to approximately 0.03 weight percent water.

| **Step** | **Setting** |
|---|---|
| **Resin Drying Process** | |
| Drying Temp | Vacuum (PEO) |
| Drying Time | |

| **Feeder Parameters** | |
|---|---|
| PEO (7,000,000 MW) | 56 grams/minute |
| HDPE | 104 grams/minute |

| **Extruder Parameters** | |
|---|---|
| Zone #1 | 375°F |
| Zone #2 | 430°F |
| Zone #3 | 450°F |
| Zone #4 | 450°F |
| Die (#5) | 450°F |
| **Screw Speed** | 134 rpm |

| **Extruder Responses** | |
|---|---|
| Die Pressure | 490 psi |
| Torque | 45% |
| Output | 20 lbs./hr |
| **Melt Temp.** | 456°F |

| **Process Water Temps** | |
|---|---|
| Chiller Temp | 60°F |
| Fluid Temp | 50°F |

| **Pelletizing Process** | |
|---|---|
| Cutter Speed | 190 (rpm) |

Example 8

A PEO with a molecular weight of 7,000,000 was mixed with Pebax 72D. The final hydrophilic polymer blend included PEO at 35% by weight. Both materials were dried under a vacuum to approximately 0.03 weight percent water.

| **Step** | **Setting** |
|---|---|
| **Resin Drying Process** | |
| Drying Temp | Vacuum |
| Drying Time | |

| **Feeder Parameters** | |
|---|---|
| PEO (7,000,000 MW) | 84 grams/minute |
| Pebax | 157 grams/minute |

| **Extruder Parameters** | |
|---|---|
| Zone #1 | 320°F |
| Zone #2 | 335°F |
| Zone #3 | 385°F |
| Zone #4 | 360°F |
| Die (#5) | 360°F |
| **Screw Speed** | 175 rpm |

| **Extruder Responses** | |
|---|---|
| Die Pressure | 510 psi |
| Torque | 87% |
| Output | 30 Ibs./hr |
| **Melt Temp.** | 360°F |

| **Process Water Temps** | |
|---|---|
| Roll Temp | 50°F |
| Chiller Temp | 74°F |

| **Pelletizing Process** | |
|---|---|
| Cutter Speed | 290 (rpm) |

Example 9

In the ninth example a PEO with a molecular weight of 7,000,000 was utilized as the hydrophilic polymer and HDPE was the structural polymer. The final hydrophilic polymer blend included PEO at 40% by weight. The PEO was dried under a vacuum to approximately 0.03 weight percent water. The produced strands were cooled by conventional means, which included running the strands through a water bath at approximately room temperature and then pelletized.

| **Step** | **Setting** |
|---|---|
| **Resin Drying Process** | |
| Drying Temp | Vacuum |
| Drying Time | |

| **Feeder Parameters** | |
|---|---|
| PEO (7,000,000 MW) | 7.4 grams/minute |
| HDPE | 4.8 grams/minute |

| **Extruder Parameters** | |
|---|---|
| Zone #1 | 375°F |
| Zone #2 | 422°F |
| Zone #3 | 431°F |
| Zone #4 | 401°F |
| Die (#5) | 400°F |
| **Screw Speed** | 175 rpm |

| **Extruder Responses** | |
|---|---|
| Die Pressure | 530 psi |
| Torque (%) | 80% |
| Output (#/hr) | 12lbs./hr |
| **Water Bath Temp** | Room Temp Bath |

Example 10

A PEO with a molecular weight of 7,000,000 was utilized as the hydrophilic polymer and Pebax 72D was the structural polymer. The final hydrophilic polymer blend included PEO at 40% by weight. The produced strands were cooled by running on a conveyor belt cooled with an air conditioner and then pelletized.

| **Step** | **Setting** |
|---|---|
| **Resin Drying Process** | |
| Drying Temp | 200° for Pebax/Vacuum for PEO |
| Drying Time | 12 hrs /12 hrs |

| **Feeder Parameters** | |
|---|---|
| PEO | 6 grams/minute |
| Pebax | 4.0 grams/minute |

| **Extruder Parameters** | |
|---|---|
| Zone #1 | 350°F |
| Zone #2 | 400°F |
| Zone #3 | 400°F |
| Zone #4 | 380°F |
| Die (#5) | 380°F |

| **Conveyance Settings** | |
|---|---|
| Screw Speed | 200 |

| **Extruder Responses** | |
|---|---|
| Die Pressure | 390 psi |
| Torque | 90% |
| Output | 9.99 lbs./hr |
| **Belt Temp** | AC cooling - forced air |

Example 11

The hydrophilic polyblend was formed utilizing 20% by weight PEO, with a molecular weight of 7,000,000, with 80% by weight Pellethane 90A. The two polymers were first mixed together with about 2% by weight triallyl triazine trione (Aldrich Chemical Co., Milwaukee, WI) and about 0.2% by weight Irganox 1098. The materials were added to the extruder through one feeder.

| **Step** | **Setting** |
|---|---|
| **Resin Drying Process** | |
| Drying Temp | PEO - 78°F with vacuum Pellethane 150°F with forced air desiccant |

| **Feeder Parameters** | |
|---|---|
| All materials | 60 grams/minute |

| **Extruder Parameters** | |
|---|---|
| Zone #1 | 335°F |
| Zone #2 | 355°F |
| Zone #3 | 365°F |
| Zone #4 | 365°F |
| Die (#5) | 380°F |

| **Conveyance Setting** | |
|---|---|
| Screw Speed | 94 rpm |

| **Extruder Responses** | |
|---|---|
| Die Pressure | 141 psi |
| Torque | 15.8% |
| **Process Temps** | Belt cooled with A/C |

| **Pelletizing Process** | |
|---|---|
| Cutter Speed | 167 (rpm) |

Comparison Example

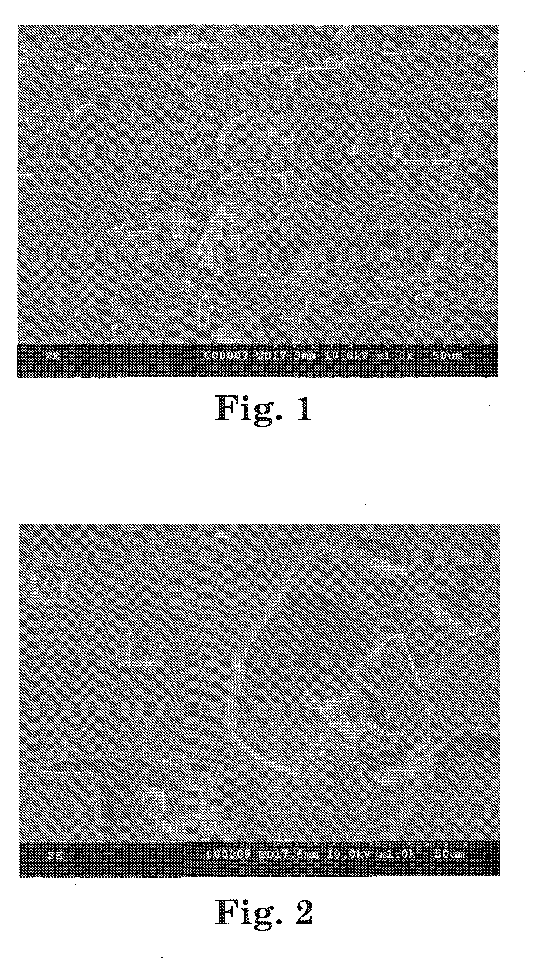

The miscibility characteristics of PEO in Pebax versus PVP in Pebax were compared. A polyblend of 40% PEO (WSR N12K, 1,000,000 MW)/60% Pebax 72D with 0.2 pphr (parts per hundred resin) Irganox B225 was formulated to compare with a polyblend of 40% PVP (K-90, 900,000 - 1,700,000 MW)/60% Pebax 72D with 0.2 pphr Irganox B225. After creation of each polyblend the material was extruded into a cylinder and cross-sectioned. The samples were placed in water at room temperature for 8 hours to dissolve the hydrophilic phase. Each sample was then dehydrated for eight hours in a vacuum oven and gold coated for viewing under a scanning electron microscope. FIG. 1 illustrates the SEM of the PEO polyblend. The PEO showed consistent and uniform dispersion in the structural material. As shown, the PEO and the Pebax form a finely dispersed blend that is substantially uniform. FIG. 2 illustrates the PVP polyblend. From the image of the PVP polyblend it is apparent that the PVP droplets are not of a uniform size, are larger, and are poorly dispersed. The PEO therefore creates a more finely dispersed polyblend material. Clumps of the PVP material were visible to the unaided eye. The PVP polyblend is therefore less miscible in the polyether block amide structural polymer and presents a less uniformly lubricious surface. In addition, large irregular deposits of PVP on the surface make the polyblend brittle and thus easier to break off during use and may be possibly released as a contaminant into the body.

Each of the co-extruded PEO and PVP polyblends were also tested for suitability in a medical device. The PVP polyblend displayed brittle characteristics and could only be elongated less than 5%. The brittleness of the compound rendered it unacceptable for medical device applications. Moreover, upon hydration in 37°C water the co-extruded PVP became only moderately lubricious to the hand.

In comparison, the PEO polyblend rendered a tough compound with high strength. The PEO polyblend was tested and showed elongation values greater than 80%. The tough properties of the PEO polyblend also made it useful for medical device applications. Moreover, upon hydration in 37°C water the PEO polyblend became very lubricious to the hand. PEO polyblends, therefore, are superior to PVP polyblends for medical device lubricating applications.

Medical Devices Formed From the Polyblend

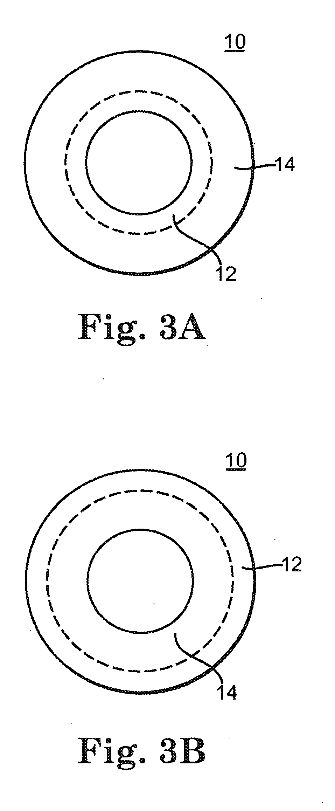

FIG. 3A illustrates a dual layer tube 10 with an inner layer 12 formed from the PEO polyblend and a polymer outer layer 14. The tube 10 may be formed by co-extrusion and the inner layer 12 may be between about 0.001-.0025 inches thick. Furthermore, the tube 10 may be incorporated into any medical device, such as, for example, a catheter, a sheath, a stent or lead delivery device, an introducer, or a dilator. The tube 12 may further be made from any of the PEO polyblend materials previously discussed. As illustrated in FIG. 3B, the outer layer 14 may be the lubricious polyblend.

The inner layer 12 forms a lubricious surface when exposed to blood or other suitable polar liquids, such as water. The lubricious surface may reduce the drag friction experienced by a lead or stent when passed through the tube. In addition, the lubricious surface may reduce drag friction when incorporated as part of a telescoping dual catheter arrangement. In this telescoping embodiment, the lubricious layer may be included on the inside of the outer guide, the outside of the inner guide, or both.

In further embodiments, the inner layer 12 (or other medical device formed utilizing the lubricious polymer blend) may be electron beam cross-linked with, for example, a 10 kEV electron beam. Such an electron beam may be at a variety of strengths, such as at 5 or 10 Mrad, and may be applied once, twice, or more than twice. Utilizing such an electron beam is known in the art for sterilizing catheters and also for helping to secure the layers of the catheter together. Cross-linking the inner layer 12 improves the retention rate of the PEO when a stent or other device is passed through the lumen of the catheter. Such cross-linking may also improve the retention rate of the lubricious surface on the outside of the catheter. Cross-linking may furthermore reduce the swell rate.

The cross-linking of the PEO polyblend may form a cross-linked polymer matrix (an interpenetrating cross-linked network) that is water swellable. Such a material may form a hydrogel when hydrated. During hydration the hydrogel will not dissolve in the aqueous solution but will become water swollen and lubricious. Determining the right amount of e-beam energy to expose the PEO polyblend to may be determined by minimizing the amount of free PEO that dissolves during hydration. This amount may be determined by the sol point or weight loss of the hydrogel after cross-linking, hydration, and drying. As may be appreciated, various structural polymer materials may be more or less susceptible to cross-linking in this manner. In addition, other agents may be added to the polyblend to improve the cross-linking and to affect the resultant structure.

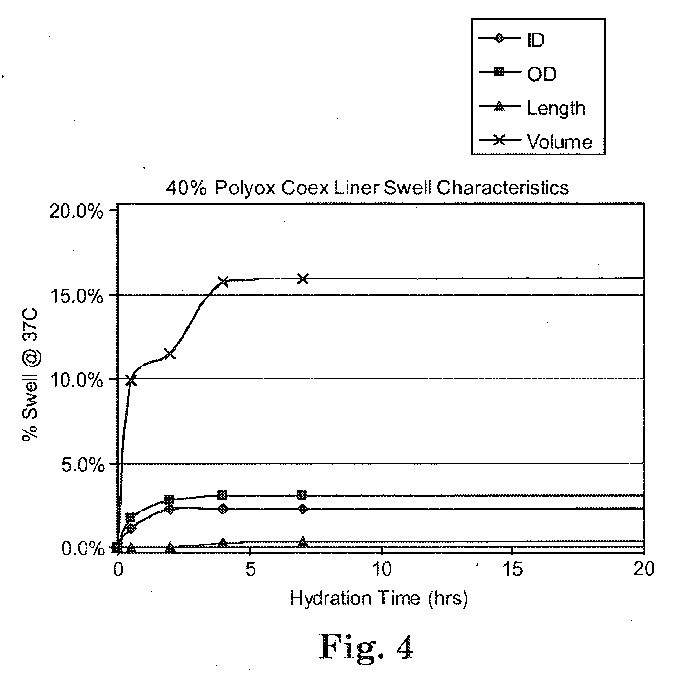

In one example, a two-layer catheter liner (tube) was formed by co-extrusion using the 40% PEO (1 million MW)/Pebax 72D polyblend as an inner layer with Nylon 12 as the outer layer. The PEO polyblend inner layer was approximately 0.002 inch thick. The swell characteristics of the liner was then determined by soaking in a water bath at 37 °C and measuring at certain time intervals. As can be seen from FIG. 4, and from the data reproduced below, after 94 hours the inner diameter of the liner had only changed about 2.2% and the outer diameter had only changed about 3.1 %.

| Hydration Time (hr) | Average ID (in) | Percent Change (%) | Average OD (in) | Percent Change (%) | Average Lenght (in) | Percent Change (%) | Average Volume (in³) | Percent Change (%) |
|---|---|---|---|---|---|---|---|---|
| 0 | 0.090 | 0.0% | 0.098 | 0.0000 | 3.000 | 0.000 | 0.0035 | 0 |
| 0.5 | 0.091 | 1.1% | 0.100 | 1.7% | 3.000 | 0.0% | 0.0039 | 9.9% |
| 2 | 0.092 | 2.2% | 0.101 | 2.8% | 3.000 | 0.0% | 0.0040 | 11.5% |
| 4 | 0.092 | 2.2% | 0.101 | 3.1% | 3.007 | 0.2% | 0.0041 | 15.7% |
| 7 | 0.092 | 2.2% | 0.101 | 3.1% | 3.010 | 0.3% | 0.0041 | 15.9% |
| 94 | 0.092 | 2.2% | 0.101 | 3.1% | 3.010 | 0.3% | 0.0041 | 15.9% |

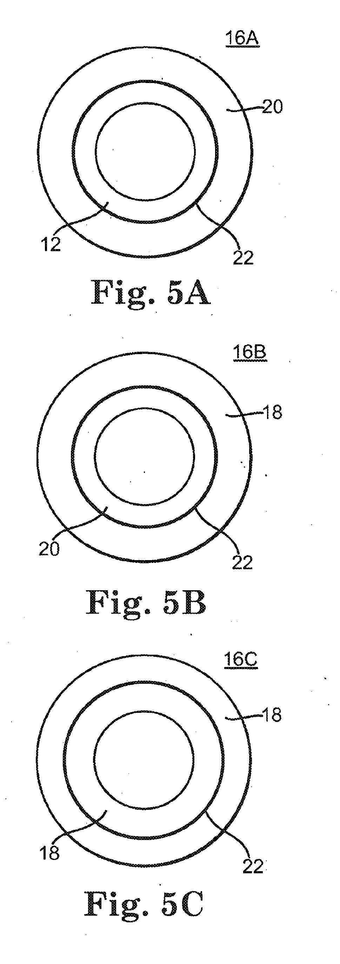

In still another embodiment illustrated in FIG. 5A, the hydrophilic polyblend may be part of a three-layer tube 16A. The lubricious polyblend may be the inner layer 18 and include another polymer as an outer layer'20. A third layer 22 formed of a third material may be disposed between the inner layer 18 and outer layer 20 to help secure them together. One such third layer 22 may include a graft maleic anhydride or an acrylic acid copolymer. In still further embodiments, as shown in FIG. 5B, the lubricious polyblend may be the outer layer 20 of a tube 16B. In yet another embodiment, as shown in FIG. 5C, both the inner layer 18 and out the outer layer 20 of a tube 16C are lubricious polyblends.

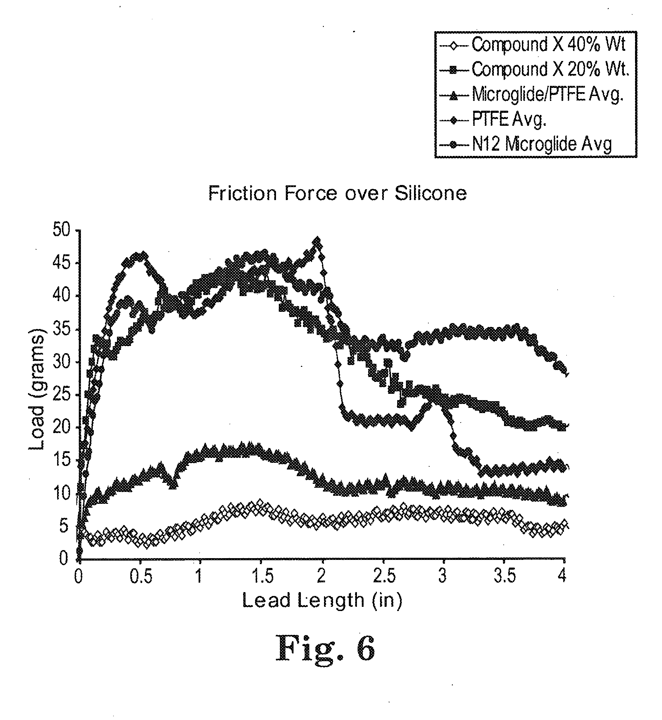

FIGS. 6-7 compare two formulations of the present invention against lubricious coatings known in the art. The polyblend was formulated to include 40% and 20% by weight PEO (1,000,000 MW) blended with Pebax 72D. The 40% PEO polyblend was made as shown in Example 1. The 20% PEO polyblend was created by diluting the 40% PEO during the extrusion process with more Pebax 72D. The testing was done by coating the interior of a catheter with the hydrophilic polyblend and using silicone and polyurethane coated leads attached to an Instron Universal Testing Machine (Canton, PA) to simulate the vascular anatomy and to measure the force necessary to pull the lead through the catheter.

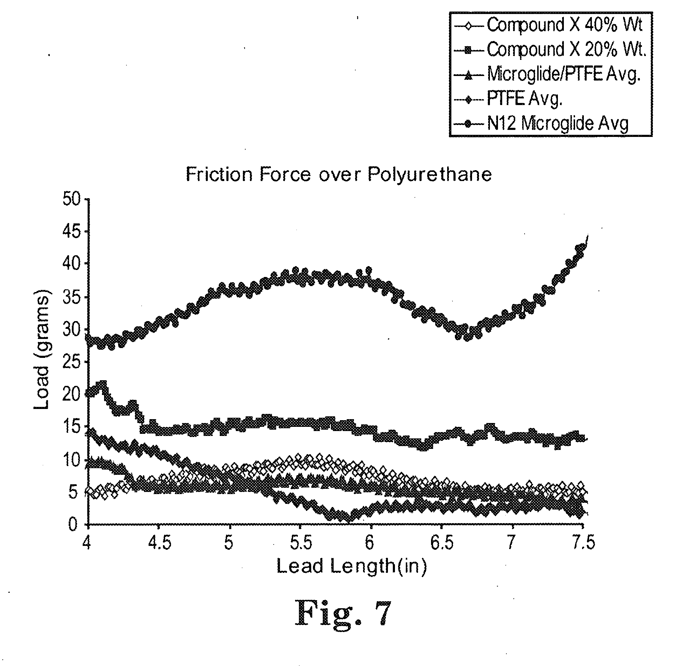

As illustrated in FIG. 6, when the present invention polyblend is utilized to form a coating the frictional force over silicone is better than three previously known compounds. The figure illustrates various catheters each separately coated with a 20% and 40% percent by weight PEO compound, a Microglide® coated PTFE, a PTFE, and a Microglide® coated nylon 12. As illustrated in FIG. 7, the frictional force over polyurethane of the present invention is better than two of the three compounds and approximately the same as the third material.

As may be appreciated, in further embodiments the lubricious polyblend of the present invention may be utilized with any type of medical device known to those in the art that benefits from a lubricious layer.

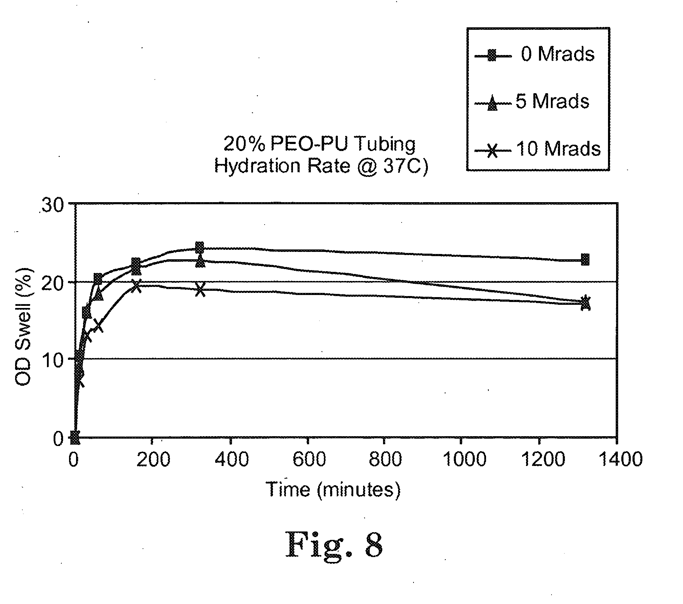

In another embodiment, a 20% PEO-PU polyblend (Pellethane® 90AE) material was formed. The material was then extruded to form a tube and cross-linked by exposure to an electron beam at 5 and 10 Mrads. As illustrated in FIG. 8, cross-linking the polyblend to form a matrix served to reduce the overall swelling of the tube when hydrated.

In further embodiments, the tubing formed from any of the previously described mixtures may be melted and utilized to directly coat a guidewire using a process such as is disclosed in U.S. Pat. No. 6,695,915, which is incorporated by reference for all that it teaches and discloses. Such a guidewire may be lubricous when exposed to blood during insertion and therefore be more easily inserted further into the vasculature. Moreover, the reduced swelling may aid in vasculature insertion. The lubricious hydrophilic polyblend may also be coated on the guidewire by other methods known to those in the art, such as dipping the guidewire directly into a melt pool of the hydrophilic polymer blend or by any other method known to those in the art. Various characteristics of the guidewire may make the polyblend may influence adhesion on to the guidewire. In further embodiments, pre-coatings or other pre-treatments may be applied to the guidewire before coating with the polyblend. Moreover, cross-linking the material coated on the guidewire may reduce swelling and improve retention of the lubricious material.

Other intravenous devices for which the present invention polyblend may impart desirable lubricious properties may include 1) a guiding catheter shaft using the hydrophilic compound as the inner layer; 2) a polymer shunt or stent delivery device where the hydrophilic compound is the inner layer and is impregnated with an anti-coagulant agent to prevent clotting, cholesterol or other blood component build up in the arteries; 3) an implantable device (lead) outer or inner layer; and 4) a lead electrode coating.

In another embodiment a 40% PEO (WSR N12K, 1,000,000 MW)/60% Pebax 72D polyblend was formed as in Example 1. The material was then extruded to form a hollow catheter tube with an inner diameter of about 0.098 inches and an outer diameter of about 0.105 inches. The lubricious qualities and the retention of the lubricious qualities of the catheter were then tested by immersing the catheter in 37°C water and inserting it into a sinusoidal passage cut into a testing block made of polycarbonate. A transducer was used to measure the force required to move a silicone tipped PU lead back and forth through the catheter while it was contained in the testing block. The testing block provides a uniform platform for the comparison of catheters and the sinusoidal shape represents curves in the vascular anatomy the catheter would be required to make during insertion into a patient.

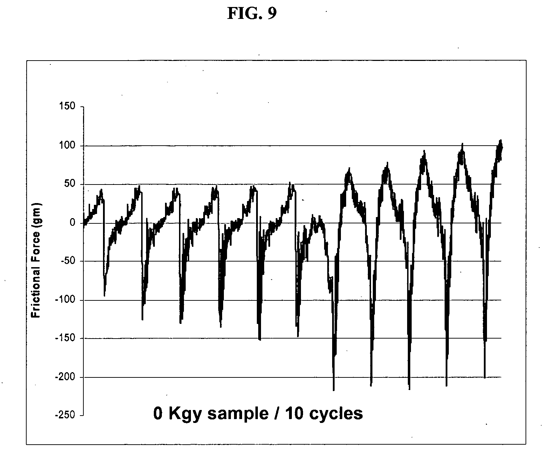

As illustrated in FIG. 9, the force required for insertion of the PU lead (+ force) and withdrawal of the lead (- force) increased over each cycle with significant increases coming after about six cycles. The increase in the required force may occur because of abrasion of the lubricious PEO from the polyblend during the passage of the PU lead. In addition, because the PEO is water soluble, some amount of the PEO may be lost through dissolution.

In an attempt to reduce the loss of the lubricious qualities, another catheter tube was formed from the same polyblend material but was exposed to 50 Kilogray (Kgy) (5 Mrad) of e-beam radiation to cross-link the polyblend. The cross-linking may occur between one or more of PEO to PEO, Pebax to Pebax, and PEO to Pebax. The last cross-linking may include the PEO grafting to the backbone of cross-linked Pebax material. The cross-linking transforms the thermoplastic material to a thermoset, fixing the polyblend into the shape it had when exposed to the radiation in addition to increasing its molecular weight. The same procedure for testing the force required to pass a PU lead through the catheter was then performed using the transducer and the testing block.

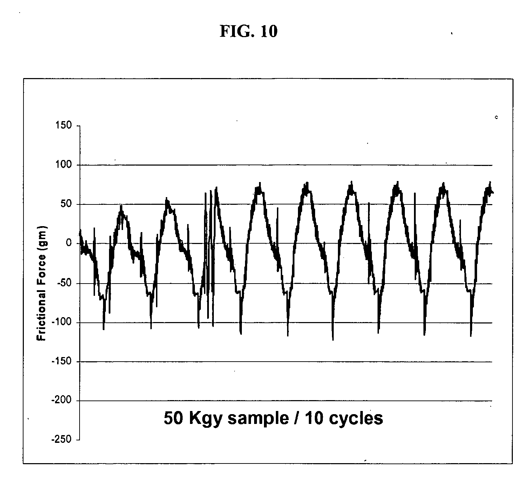

As illustrated in FIG. 10, the force required to insert and then withdraw the PU lead did not significantly increase over time as it did with the non cross-linked catheter. Instead, the insertion force generally plateaued after a few runs. The cross-linking of the polyblend therefore improved the retention of the lubricious qualities of the catheter tube. The retention of the lubricious quality is due to stopping or reducing the loss of the lubricious PEO from the polyblend.

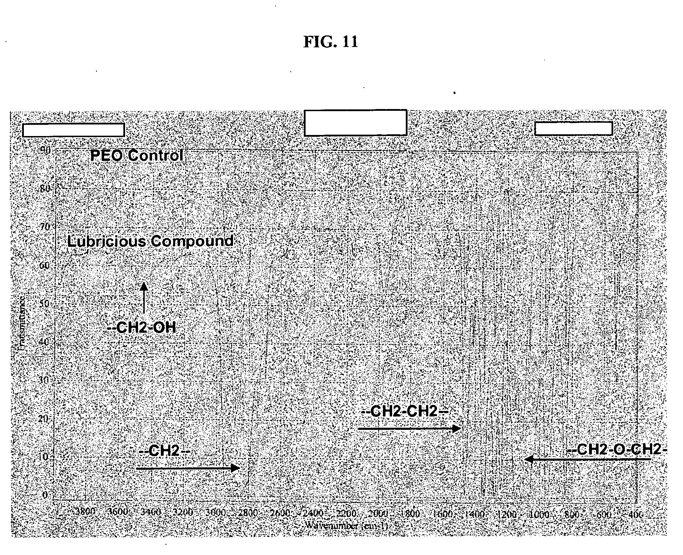

To determine whether the water was extracting the PEO from the polyblend, pellets of the PEO (WSR N12K, 1,000,000 MW)/60% Pebax 72D polyblend (non cross-linked) were soaked in 37 °C water for 48 hours. A sample of the liquid was then collected, placed on a slide and dried. The sample was tested on a Fourier Transform Infra Red (FTIR) Spectrophotometer to identify the materials in the solution. FIG. 11 indicates the spectrograph of the polyblend extract at 20. A spectrograph of a PEO solution standard is shown at 30.

Comparing the extract with the PEO standard shows that PEO was present in solution. Soaking the non cross-linked polyblend in water therefore results in the loss of the PEO from the polyblend and into solution. In addition, no Pebax was detected. It can therefore be assumed that only the PEO or substantially only the PEO is extracted by the water and that the loss of the PEO reduces the lubricious qualities of material.

To better determine the effect of the radiation upon the polyblend, additional samples of the pellets were irradiated at 1, 2.5, 5, 10 and 25 Mrad (1 Mrad = 10Kgy) and tested using some of the procedures set forth in ASTM D2765. In one test, each irradiated sample was weighed, immersed in 37 °C water for 48 hours, rinsed, dried and then weighed again. This test, called a gel point test, was used to determine the weight percentage PEO lost. In another test the irradiated polyblends were soaked for four hours in water at 37 °C and examined for cloudiness. The cloudier the solution the more PEO lost by the polyblend into solution.

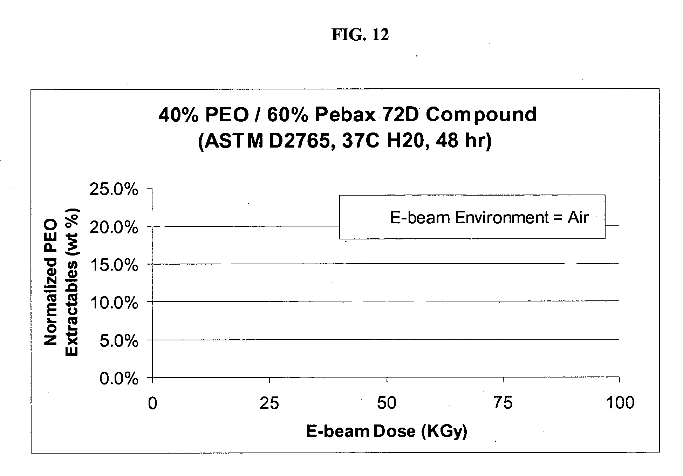

As illustrated in FIG. 12, the weight percentage of PEO extracted from the polyblend during the gel point testing was reduced for the samples irradiated with up to 5 Mrad of radiation. At some point between 5 Mrad and 10 Mrad the amount of PEO extracted by the water began to increase. It is therefore evident that cross-linking results in a polyblend with improved PEO retention capabilities, and therefore improve retention of the lubricious properties. The increase in the PEO extracted with radiation doses from between 5 Mrad and 10 Mrad may be due to scission of the polyblend. Rather than forming further cross-linked material the radiation causes the already chains to break down and therefore decrease the molecular weight and therefore causes a reduction in the retention of the PEO.

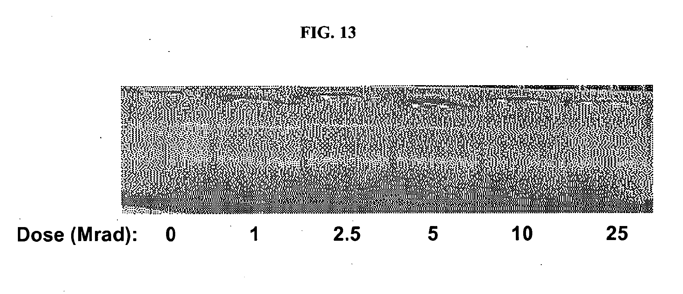

As illustrated in FIG. 13, the cloudiness of the water changed with increased radiation doses. The increased cloudiness of the solution in the beakers represents an increase in the amount of PEO extracted from the polyblend and dissolved into solution. Solutions made from soaking the polyblend materials that were exposed to 2.5 Mrad and 5.0 Mrad radiation doses produced the clearest water extract. Moreover, in agreement with the weight percentage testing, radiation exposure somewhere between 5.0 Mrad and 10.0 Mrad started to cause an increase in the cloudiness of the solution, indicating an increase in the amount of PEO extracted from the polyblend. In different embodiments, using different polyblend ratios or using different materials to form the polyblends, the amount of radiation energy required to reach an optimized cross-linking state so as to improve the lubricious polymer retention may vary.

Next, the addition of a cross-linking agent and the immersion of the polyblend in an inert atmosphere during exposure to the e-beam radiation were both examined. Four samples of the polyblend pellets were irradiated with 2.5 Mrad of radiation under a variety of conditions and compared to non-irradiated pellets. The e-beam radiation conditions included 1) a regular air atmosphere; 2) an inert argon atmosphere; 3) a regular air atmosphere with the addition of a cross-linking agent; and 4) an inert atmosphere with the addition of a cross-linking agent.

For the examples that included a cross-linking agent, about 1% by weight triallyl isocyanurate (Aldrich Chemical Co., Milwaukee, WI) was mixed with the polyblend material, in the present embodiment, the cross-linking agent was added during the formation of the polyblend. The addition of the cross-linking agent during formation and mixing of the polyblend also resulted in improved mixing because the cross-linking agent improved the flow properties of the materials. In further embodiments the cross-linking agent can be added during any step desired and that is compatible with the cross-linking agent and the polyblend.

In order to expose the various polyblend materials to the desired atmosphere during cross-linking, the polyblend pellets were first placed in a foil lined pouch. The atmosphere in the pouch was then expunged, replaced with the desired inert gas or gas mixture, and sealed. Each of the five pellet samples were then weighed, soaked in water at 37 °C for 48 hours, dried, and then weighed again.

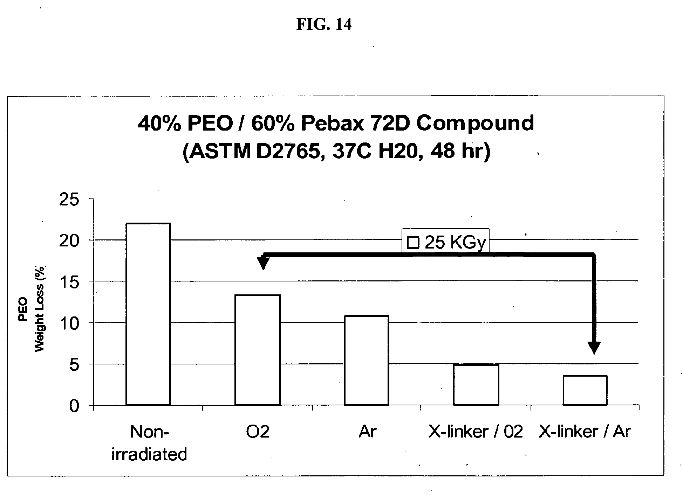

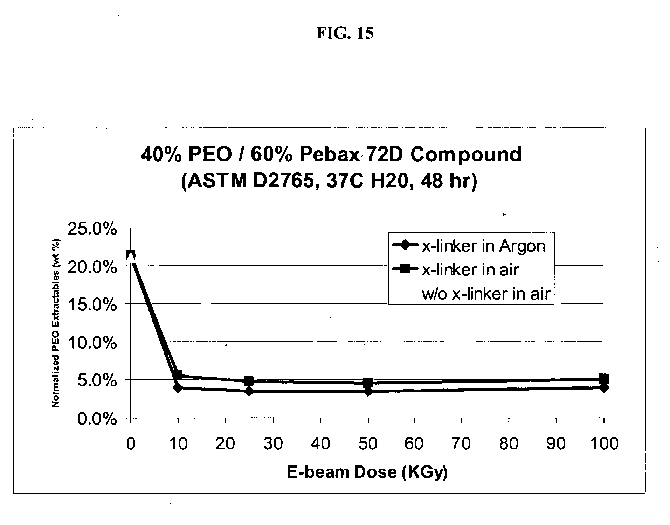

As illustrated in FIGS. 14-15, the percent weight loss of material was reduced by cross-linking and reduced even further by cross linking in an inert atmosphere. Moreover, the addition of the cross-linking agent caused an even greater reduction in the amount of PEO lost. The addition of the cross-linking agent and exposure to radiation in a normal atmosphere reduced the PEO loss even more than cross-linking in just the inert atmosphere (without the cross-linking agent). The combination of an inert atmosphere and use of a cross-linking agent provided polyblend pellets with the lowest PEO loss of the samples tested.

The testing discussed above shows that e-beam cross-linking is an effective way to improve the retention of the water soluble lubricious component of the polyblend. The loss of lubricity for medical devices made from the cross-linked polyblend may therefore be reduced. Furthermore, the addition of a cross-linking agent and performing the cross-linking in an inert atmosphere enhances the effectiveness of cross-linking the polyblend. The reduction in the loss of PEO from the polyblend will correspond to a reduction in the loss of lubricity.

In further embodiments the cross-linking agents may include other materials, such as, for example, multi-functional allylic materials like triallyl cyanurate, diallyl cyclohexane, 1,7 octadiene, 1,9 decadiene, and 2,4,6 triallyloxy-1,3,5 triazine. In other embodiments other cross-linking agents may be used depending on the specific polyblend formulation utilized. In still further embodiments the inert atmosphere may include other inert gases, such as, for example, nitrogen. In additional embodiments the radiation exposure may be more or less depending on the needs of the polyblend and may include up to or more than 25 Mrad.

The cross-linking of other polyblend materials, including, but not limited to, the other polyblend materials discussed above, may also reduce the loss of lubricity due to abrasion or extraction of the lubricious polymer from the polyblend. Such materials may include PEO with another structural polymer, such as HDPE, natural and synthetic rubbers, styrenics, thermoplastic elastomers, Nylons, polyurethanes other specialty polymers, etc. Other lubricious polymers may include, for example, PPO, EVOH, EVA, polyoxymethylene, and PVP.

The cross-linked polyblend can be utilized to make or coat any type of medical device or other device where inclusion of a lubricious surface is desired. The polyblend can be utilized to make catheters, sheaths, stents or lead delivery devices, introducers, dilators, etc. In addition, the polyblend may utilized to coat surfaces on these or other medical devices, such as on guidewires, wherein a lubricous surface is desired. Moreover, the polyblend may be further utilized to release a biologically active agent from the polyblend.

Various modifications and additions can be made to the exemplary embodiments discussed without departing from the scope of the present invention. Accordingly, the scope of the present invention is intended to embrace all such alternatives, modifications, and variations as fall within the scope of the claims, together with all equivalents thereof.

## Claims

1. A method of forming a medical device from a lubricious polymer comprising:
drying a polyethylene oxide of a molecular weight between about 200,000 and about 7,000,000;
drying a polyether block amide;
melt mixing the polymers into a generally uniform blend by feeding the polymers at a desired rate into a compounding extruder;
forming the blend into a desired shape; and cross-linking the polymer blend

2. The method of claim 1 wherein cross-linking the polymer further comprises applying radiation to the polymer.

3. The method of claim 1 further comprising adding a cross-linking agent to the blend while melt mixing the polymers

4. The method of claim 3 wherein the cross-linking agent is a multi-functional allylic compound.

5. The method of claim 4 wherein the multi-functional allylic compound is one or more of the group comprising triallyl isocyanurate, triallyl cyanurate, triallyl cyclohexane, 1,7 octadiene, 2,4,6 triallyloxy-1,3,5 triazine and 1,9 decadiene.

6. The method of claim 1 wherein cross linking the polymer blend includes exposing the medical device to between about 0.0 and about 5.0 Mrad of radiation.

7. The method of claim 1 wherein cross linking the polymer blend includes exposing the medical device to between about 0.0 and about 10.0 Mrad of radiation.

8. The method of claim 1 further comprising placing the medical device in an inert atmosphere and then exposing the medical device to the desired amount of radiation.

9. The method of claim 8 wherein the inert atmosphere is comprised of one or more of the group consisting of argon and nitrogen.

10. The method of claim 1 wherein drying the polyethylene oxide comprises drying a polyethylene oxide with a molecular weight of about 1,000,000.

11. The method of claim 1 wherein forming the blend into a desired shape further comprises integrating the blend into a medical device.

12. The method of claim 1 wherein the polyethylene oxide and the polyether block amide are in a ration to produce a polymer that is about 40 % polyethylene.

13. A lubricious polymer blend comprising:
a finely dispersed blend of a polyethylene oxide of a molecular weight between about 200,000 and about 7,000,000 and a polyether block amide, the blend including u p to about 60% by weight of the polyethylene oxide and about 1% of a cross-linking agent, wherein after being formed into a desired shape the blend is cross-linked sufficiently to substantially reduce the loss of the polyethylene oxide due to dissolution and abrasion.

14. The lubricious polymer blend of claim 13 wherein the polymer blend is cross-linked in the presence of an inert atmosphere.

15. The lubricious polymer blend of claim 13 wherein the polymer blend is cross-linked by exposure to a desired amount of radiation.

16. The lubricious polymer blend of claim 13 wherein the polymer blend is exposed to between about 0.0 and about 5.0 Mrad of radiation.

17. The lubricious polymer blend of claim 13 wherein the polymer blend is exposed to between about 0.0 and about 10.0 Mrad of radiation.

18. The lubricious polymer blend of claim 13 wherein the cross-linking agent is a multi-functional allylic compound.

## Patentansprüche

1. Verfahren zur Herstellung eines medizinischen Instrumentes aus einem Schmierstoff-Polymer umfassend:
Trocknung eines Polyethylenoxids mit einem Molekulargewicht zwischen etwa 200.000 und etwa 7.000.000,
Trocknung eines Polyetherblockamids,
Schmelzmischung des Polymers in einen hauptsächlich gleichförmigen Blend durch Einspeisung des Polymers in einen Misch-Extruder mit einer gewünschten Rate,
Ausformen des Blends in eine gewünschte Form und Quervernetzung des Polymerblends.

2. Verfahren nach Anspruch 1, **dadurch gekennzeichnet, dass** die Quervernetzung des Polymers zusätzlich die Anwendung einer Bestrahlung des Polymers umfasst.

3. Verfahren nach Anspruch 1, das weiterhin den Zusatz eines Quervernetzungsmittels zum Blend während der Schmelzmischung umfasst.

4. Verfahren nach Anspruch 3, **dadurch gekennzeichnet, dass** das Quervernetzungsmittel eine multi-funktionelle allylische Verbindung ist.

5. Verfahren nach Anspruch 4, **dadurch gekennzeichnet, dass** die multi-funktionelle allylische Verbindung eine oder mehrere aus der Gruppe enthaltend Triallylisocyanurat, Triallylcyanurat, Triallylcyclohexan, 1,7-Octadien, 2,4,6-Triallyloxy-1,3,5-triazin und 1,9-Decadien ist.

6. Verfahren nach Anspruch 1, **dadurch gekennzeichnet, dass** bei der Quervernetzung des Polymerblends das medizinische Instrument einer Strahlung zwischen etwa 0,0 und etwa 5,0 Mrad ausgesetzt wird.

7. Verfahren nach Anspruch 1, **dadurch gekennzeichnet, dass** bei der Quervernetzung des Polymerblends das medizinische Instrument einer Strahlung zwischen etwa 0,0 und etwa 10,0 Mrad ausgesetzt wird.

8. Verfahren nach Anspruch 1, das weiterhin die Lagerung des medizinischen Instrumentes in einer inerten Atmosphäre umfasst und anschließend das medizinische Instrument der gewünschten Strahlungsmenge ausgesetzt wird.

9. Verfahren nach Anspruch 8, **dadurch gekennzeichnet, dass** die inerte Atmosphäre eine oder mehrere aus der Gruppe bestehend aus Argon und Stickstoff enthält.

10. Verfahren nach Anspruch 1, **dadurch gekennzeichnet, dass** die Trocknung des Polyethylenoxids die Trocknung eines Polyethylenoxids mit einem Molekulargewicht von etwa 1.000.000 umfasst.

11. Verfahren nach Anspruch 1, **dadurch gekennzeichnet, dass** die Ausformung des Blends in eine gewünschte Form weiterhin die Integration des Blends in ein medizinisches Instrument umfasst.

12. Verfahren nach Anspruch 1, **dadurch gekennzeichnet, dass** das Polyethylenoxid und das Polyetherblockamid in einem Verhältnis vorliegen, dass ein Polymer mit etwa 40 % Polyethylen erhalten wird.

13. Schmierstoff-Polymerblend umfassend:
ein fein dispergiertes Blend eines Polyethylenoxids mit einem Molekulargewicht zwischen etwa 200.000 und etwa 7.000.000 und ein Polyetherblockamid, das Blend bis zu etwa 60 Gew.-% des Polyethylenoxids und etwa 1 % eines Quervernetzungsmittels enthält, wobei nach der Ausformung in die gewünschte Form das Blend ausreichend quervernetzt ist, dass der Verlust des Polyethylenoxids aufgrund von Auflösung und Abrieb wesentlich reduziert ist.

14. Schmierstoff-Polymerblend nach Anspruch 13, **dadurch gekennzeichnet, dass** der Polymerblend in Gegenwart einer inerten Atmosphäre quervernetzt wird.

15. Schmierstoff-Polymerblend nach Anspruch 13, **dadurch gekennzeichnet, dass** das Polymerblend bei Bestrahlung mit einer gewünschten Strahlungsmenge quervernetzt wird.

16. Schmierstoff-Polymerblend nach Anspruch 13, **dadurch gekennzeichnet, dass** das Polymerblend einer Strahlung zwischen etwa 0,0 und etwa 5,0 Mrad ausgesetzt wird.

17. Schmierstoff-Polymerblend nach Anspruch 13, **dadurch gekennzeichnet, dass** das Polymerblend einer Strahlung zwischen etwa 0,0 und etwa 10,0 Mrad ausgesetzt wird.

18. Schmierstoff-Polymerblend nach Anspruch 13, **dadurch gekennzeichnet, dass** das Quervernetzungsmittel eine multi-funktionelle allylische Verbindung ist.

## Revendications

1. Procédé de formation d'un dispositif médical à partir d'un polymère lubrifiant comprenant :
le séchage d'un oxyde de polyéthylène d'un poids moléculaire entre environ 200.000 et environ 7.000.000 ;
le séchage d'un polyéther bloc amide ;
le mélange en fusion des polymères selon un mélange généralement uniforme en alimentant les polymères selon un débit souhaité dans une extrudeuse mélangeuse ;
la conformation du mélange selon une forme souhaitée ; et la réticulation du mélange de polymères.

2. Procédé selon la revendication 1, dans lequel la réticulation du polymère comprend en outre l'application d'un rayonnement au polymère.

3. Procédé selon la revendication 1, comprenant en outre l'ajout d'un agent de réticulation au mélange pendant le mélange en fusion des polymères.

4. Procédé selon la revendication 3, dans lequel l'agent de réticulation est un composé allylique multifonctionnel.

5. Procédé selon la revendication 4, dans lequel le composé allylique multifonctionnel est un ou plusieurs parmi le groupe comprenant isocyanurate de triallyle, cyanurate de triallyle, cyclohexane de triallyle, 1,7 octadiène, 2,4,6 triallyloxy-1,3,5 triazine et 1,9 décadiène.

6. Procédé selon la revendication 1, dans lequel la réticulation du mélange de polymères inclut l'exposition du dispositif médical à entre environ 0,0 et environ 5,0 Mrad de rayonnement.

7. Procédé selon la revendication 1, dans lequel la réticulation du mélange de polymères inclut l'exposition du dispositif médical à entre environ 0,0 et environ 10,0 Mrad de rayonnement.

8. Procédé selon la revendication 1, comprenant en outre le placement du dispositif médical dans une atmosphère inerte puis l'exposition du dispositif médical à la quantité de rayonnement souhaitée.

9. Procédé selon la revendication 8, dans lequel l'atmosphère inerte est composée d'un ou plusieurs éléments du groupe constitué par argon et azote.

10. Procédé selon la revendication 1, dans lequel le séchage de l'oxyde de polyéthylène comprend le séchage d'un oxyde de polyéthylène avec un poids moléculaire d'environ 1.000.000.

11. Procédé selon la revendication 1, dans lequel la conformation du mélange selon une forme souhaitée comprend en outre l'intégration du mélange dans un dispositif médical.

12. Procédé selon la revendication 1, dans lequel l'oxyde de polyéthylène et le polyéther bloc amide sont selon un rapport pour produire un polymère qui est du polyéthylène à environ 40%.

13. Mélange de polymères lubrifiant comprenant :
un mélange finement dispersé d'un oxyde de polyéthylène d'un poids moléculaire entre environ 200.000 et environ 7.000.000 et d'un polyéther bloc amide, le mélange incluant jusqu'à environ 60% en poids de l'oxyde de polyéthylène et environ 1 % d'un agent de réticulation, dans lequel, après avoir été conformé selon une forme souhaitée, le mélange est réticulé suffisamment pour réduire substantiellement la perte de l'oxyde de polyéthylène due à la dissolution et à l'abrasion.

14. Mélange de polymères lubrifiant selon la revendication 13, dans lequel le mélange de polymères est réticulé en présence d'une atmosphère inerte.

15. Mélange de polymères lubrifiant selon la revendication 13, dans lequel le mélange de polymères est réticulé par exposition à une quantité de rayonnement souhaitée.

16. Mélange de polymères lubrifiant selon la revendication 13, dans lequel le mélange de polymères est exposé à entre environ 0,0 et environ 5,0 Mrad de rayonnement.

17. Mélange de polymères lubrifiant selon la revendication 13, dans lequel le mélange de polymères est exposé à entre environ 0,0 et environ 10,0 Mrad de rayonnement.

18. Mélange de polymères lubrifiant selon la revendication 13, dans lequel l'agent de réticulation est un composé allylique multifonctionnel.
